# EUROPEAN PATENT APPLICATION

(11) **EP 4 567 025 A1**
(43) Date of publication of application: **11.06.2025**
(21) Application number: 23849906.5
(22) Date of filing: 20.07.2023
(51) Int. Cl.: C07C 309/66, C07C 303/28, C07C 309/73, C08G 77/48, C08G 77/60

(54) **FLUORENE COMPOUND HAVING SULFONIC ACID ESTER STRUCTURE AND ALLYL GROUP AND METHOD FOR PRODUCING SAME**

(30) Priority: 03.08.2022 JP 2022123934
(71) Applicant: SHIN-ETSU CHEMICAL CO., LTD., Tokyo 1000005 (JP)
(72) Inventor: OMORI, Hiroto, Annaka-shi Gunma 379-0224 (JP)
(74) Representative: Ter Meer Steinmeister & Partner
(86) International application number: PCT/JP2023/026540
(87) International publication number: WO 2024/029351

(57) **Abstract**

Provided is a fluorene compound having a sulfonic acid ester structure and an allyl group, the compound being represented by formula (1). 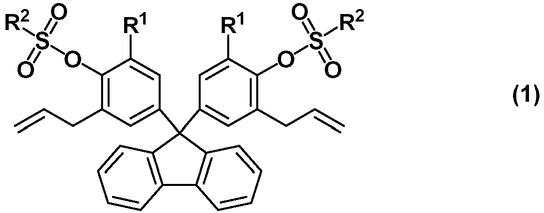 (In the formula, R¹ each independently are a hydrogen atom or a methyl group. R² each independently are a C1-20 hydrocarbyl group, some or all of the hydrogen atoms of which may be substituted by a halogen atom, a nitro group, or a cyano group, and an ester bond or an ether bond may be present between the carbon-carbon bonds thereof.)

## Description

### TITLE OF INVENTION

### FLUORENE COMPOUND HAVING SULFONIC ACID ESTER STRUCTURE AND ALLYL GROUP AND METHOD FOR PRODUCING SAME

### TECHNICAL FIELD

This invention relates to a fluorene compound having sulfonic acid ester structures and allyl groups and a method for preparing the same.

### BACKGROUND ART

As one of prior art fluorene compounds having allyl groups, Patent Document 1 describes the compound having allyl and epoxy groups, represented by the following formula (X), which is known as a functional epoxy resin. It is ascertained that the epoxy resin having a fluorene skeleton introduced therein is improved in heat resistance and electrical properties over prior art epoxy resins.

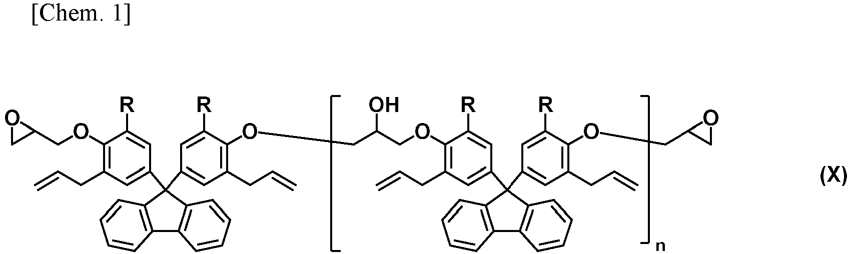

Since the introduction of fluorene skeletons into resins is effective for improving heat resistance and electrical properties, fluorene compounds are important for the development of various functional resins. Inter alia, fluorene compounds having allyl groups are not only easy to handle, but are also quite important in that polymers can be synthesized through reactions beginning with allyl groups, typically hydrosilation reaction.

As the fluorene compounds having allyl groups, Patent Documents 1 to 3 describe compounds having a phenolic hydroxy group and compounds having a glycidyl form of phenolic hydroxy group. Organosilicon compounds are synthesized by hydrosilation of these compounds, but are undesirably susceptible to oxidation and colored upon light irradiation.

### PRIOR ART DOCUMENTS

### PATENT DOCUMENTS

Patent Document 1: JP 4873223
Patent Document 2: JP-A 2014-047207
Patent Document 3: JP-A 2014-062055

### SUMMARY OF INVENTION

### TECHNICAL PROBLEM

An object of the invention, which has been made under the above-mentioned circumstances, is to provide a fluorene compound having sulfonic acid ester structures and allyl groups which is a starting material from which a fluorene skeleton-containing organosilicon compound having excellent light resistance can be synthesized by introducing a sulfonyl group as a strongly electron-attractive substituent to impart high oxidation resistance, and a method for preparing the same.

### SOLUTION TO PROBLEM

Making extensive investigations to attain the above object, the inventor has found that the outstanding problems can be solved by a fluorene compound having specific sulfonic acid ester structures and allyl groups. The invention is predicated on this finding.

The invention provides a fluorene compound containing sulfonic acid ester structures and allyl groups and a method for preparing the same, as defined below.
1. A fluorene compound having sulfonic acid ester structures and allyl groups, represented by the formula (1): 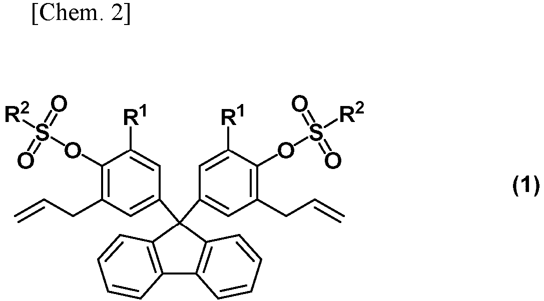 wherein R¹ is each independently hydrogen or methyl and
   R² is each independently a C1-C20 hydrocarbyl group in which some or all of the hydrogen atoms may be substituted by halogen, nitro or cyano, and an ester bond or ether bond may intervene in a carbon-carbon bond.
2. The fluorene compound of 1 wherein both R¹ are hydrogen.
3. The fluorene compound of 1 or 2 wherein both R² have 1 to 8 carbon atoms.
4. A method for preparing a fluorene compound represented by the formula (1), comprising the step of reacting a fluorene compound having allyl groups, represented by the formula (2), with a compound represented by the formula (3) or (4), 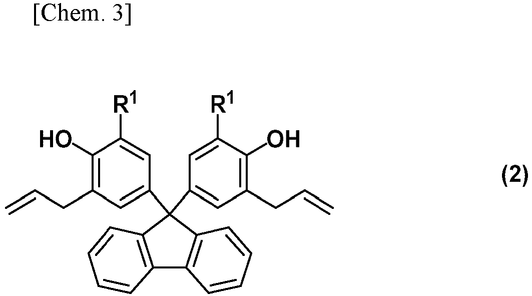 wherein R¹ is each independently hydrogen or methyl, 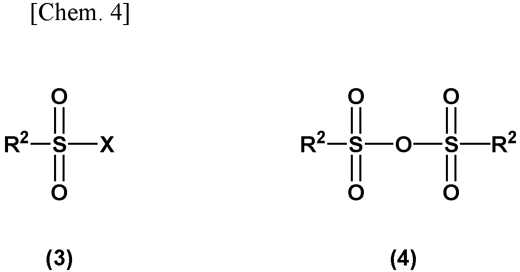 wherein R² is each independently a C1-C20 hydrocarbyl group in which some or all of the hydrogen atoms may be substituted by halogen, nitro or cyano, and an ester bond or ether bond may intervene in a carbon-carbon bond, and X is fluorine, chlorine or bromine, 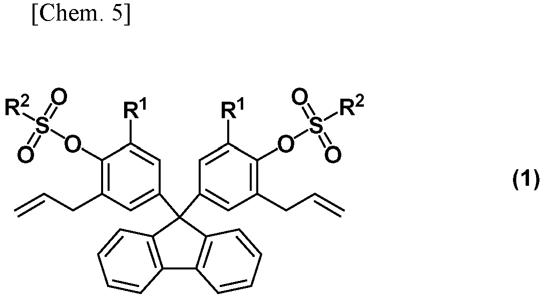 wherein R¹ and R² are as defined above.
5. A method for preparing a fluorene skeleton-containing organosilicon compound comprising the step of effecting hydrosilation reaction of the fluorene compound of any one of 1 to 3 with an organosilicon compound containing a Si-H group in the presence of a catalyst.

### ADVANTAGEOUS EFFECTS OF INVENTION

Since the sulfonyl group is strongly electron attractive, the fluorene compound containing sulfonic acid ester structures and allyl groups according to the invention is useful in that a fluorene skeleton-containing organosilicon compound is obtained from hydrosilation of the fluorene compound. It is expected that the organosilicon compound thus obtained is unsusceptible to coloration by oxidation as compared with organosilicon compounds obtained from prior art well-known fluorene compounds having allyl groups, and has excellent light resistance.

### DESCRIPTION OF EMBODIMENTS

One embodiment of the invention is a fluorene compound having sulfonic acid ester structures and allyl groups, represented by the formula (1), which is simply referred to as "fluorene compound," hereinafter.

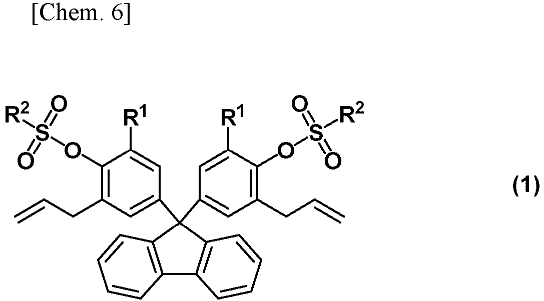

In formula (1), R¹ is each independently hydrogen or methyl, preferably hydrogen for availability of starting reactants.

In formula (1), R² is each independently a C1-C20 hydrocarbyl group in which some or all of the hydrogen atoms may be substituted by halogen (e.g., fluorine, chlorine, bromine or iodine), nitro or cyano, and an ester bond or ether bond may intervene in a carbon-carbon bond. The hydrocarbyl group may be saturated or unsaturated and straight, branched or cyclic. Examples thereof include C1-C20 alkyl groups such as methyl, ethyl, isopropyl, n-butyl, isobutyl, sec-butyl, tert-butyl, n-pentyl, n-hexyl, n-heptyl, n-octyl, n-nonyl, and n-decyl; C3-C20 cyclic saturated hydrocarbon groups such as cyclopropyl, cyclobutyl, cyclopentyl, cyclohexyl, norbornyl, and adamantyl; C2-C20 alkenyl groups such as ethenyl, 1-propenyl, 2-propenyl, 1-methyl-1-ethenyl, 1-butenyl, 2-butenyl and 3-butenyl; C2-C20 alkynyl groups such as ethynyl, 1-propynyl, 2-propynyl, 1-butynyl, 2-butynyl and 3-butynyl; C3-C20 cyclic unsaturated hydrocarbyl groups such as 1-cycloallyl, 1-cyclobutenyl, 1-cyclopentenyl, 2-cyclopentenyl, 3-cyclopentenyl, 1-cyclohexenyl, 2-cyclohexenyl and 3-cyclohexenyl; and C6-C20 aromatic ring-containing hydrocarbyl groups such as benzyl, phenyl, o-tolyl, m-tolyl, p-tolyl, mesityl, 1-naphthyl and 2-naphthyl.

In formula (1), R² is preferably a C1-C8 hydrocarbyl group for availability of starting reactants, more preferably methyl, trifluoromethyl, phenyl, 2-nitrophenyl or o-tolyl.

The fluorene compound can be synthesized by reacting a bisphenol fluorene compound having allyl groups, represented by the formula (2), with a compound represented by the formula (3) or (4). The compound represented by formula (3) or (4) may be used alone or in admixture of two or more. 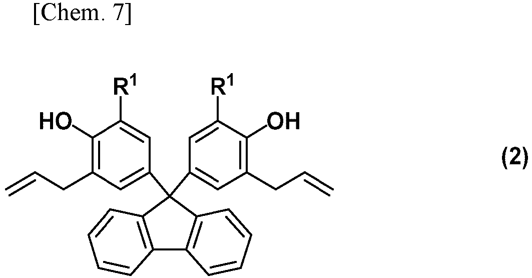 Herein R¹ is as defined above. 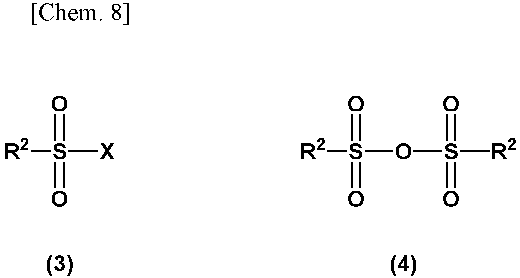 Herein R² is as defined above.

In formula (3), X is fluorine, chlorine or bromine, preferably chlorine.

For the reaction of the compound having formula (2) with the compound having formula (3) or (4), any of methods known in the art may be applied. The reaction temperature is typically 0°C to 120°C, though arbitrary, and the reaction time is typically about 2 to about 30 hours.

Although the amounts of the compound having formula (2) and the compound having formula (3) or (4) used are arbitrary, the amount of the compound having formula (3) or (4) used is typically 1.0 to 30 moles per mole of the compound having formula (2). The amount of the compound having formula (3) or (4) is preferably 2.0 to 20 moles, more preferably 2.5 to 15 moles per mole of the compound having formula (2) because the compound having formula (1) is obtained in high yields.

In the reaction, a base may be added. Examples of the base include metal hydroxides such as sodium hydroxide and potassium hydroxide, metal carbonates such as sodium carbonate and potassium carbonate, secondary or tertiary amines such as diethylamine, triethylamine, and diisopropylethylamine, pyridine and dimethylaminopyridine. The amount of the base used is preferably 2 to 15 moles per mole of the compound having formula (2). The base may be used alone or in admixture of two or more.

In the reaction, a solvent may be used. Although the solvent is not particularly limited, aprotic polar solvents are preferred. Suitable aprotic polar solvents include methylene chloride, tetrahydrofuran, acetone, acetonitrile, dimethylformamide, dimethyl sulfoxide, and 1,4-dioxane. The amount of the solvent used is typically 30 to 500 parts by weight, preferably 50 to 300 parts by weight per 100 parts by weight of the compound having formula (2).

The reaction product resulting from the above reaction is washed with water and heated under reduced pressure to remove the solvent or the like, obtaining the desired fluorene compound. In the washing step, any of aqueous solutions of metal hydroxides such as sodium hydroxide and potassium hydroxide and metal carbonates or hydrogencarbonates such as sodium carbonate, sodium hydrogencarbonate and potassium carbonate may be used.

For utilization of allyl groups, the fluorene compound can be subjected to hydrosilation reaction with an organosilicon compound having a Si-H group. Although the amounts of the fluorene compound and the SiH-containing organosilicon compound used in the hydrosilation reaction are not particularly limited, 0.9 to 0.99 mole of the SiH-containing organosilicon compound is preferably used per mole of the fluorene compound. A polymer having a high molecular weight is then obtained.

Examples of the SiH-containing organosilicon compound include, but are not limited to, hydrosilanes such as trimethoxyhydrosilane, triethoxyhydrosilane and triethylsilane; dimethylpolysiloxanes containing Si-H groups at both ends such as 1,1,3,3-tetramethyldisiloxane, α,ω-dihydrodimethylpolysiloxane and α,ω-dihydromethylphenylpolysiloxane; dimethylpolysiloxanes containing a Si-H group at one end such as 1,1,3,3,3-pentamethyldisiloxane and 1,1,1,3,3,5,5-heptamethyltrisiloxane; dimethylpolysiloxanes containing a Si-H group on side chain such as 1,1,1,3,5,5,5-heptamethyltrisiloxane; methylphenylpolysiloxanes containing a Si-H group on side chain such as 1,1,1,5,5,5-hexamethyl-3-phenyltrisiloxane; cyclic polysiloxanes containing a Si-H group such as 2,4,6-trimethylcyclotrisiloxane and 2,4,6,8-tetramethylcyclotetrasiloxane; branched polysiloxanes containing a Si-H group; methylsilicone resins containing a Si-H group; methylphenylsilicone resins containing a Si-H group; silphenylene compounds containing a Si-H group; and silalkylene compounds containing a Si-H group.

In the hydrosilation reaction of the fluorene compound with the organosilicon compound having a Si-H group, another vinyl-containing compound may be co-reacted if desired. Examples of the other vinyl-containing compound include dimethylpolysiloxanes containing vinyl groups at both ends such as 1,3-divinyl-1,1,3,3-tetramethyldisiloxane, α,ω-divinyldimethylpolysiloxane and α,ω-divinylmethylphenylpolysiloxane; dimethylpolysiloxanes containing a vinyl group on side chain such as 1,1,1,3,5,5,5-heptamethyl-3-vinyltrisiloxane; methylvinylphenylpolysiloxanes containing a vinyl group on side chain such as 1,1,1,5,5,5-hexamethyl-3-vinyl-3-phenyltrisiloxane; vinyl-containing cyclic polysiloxanes such as 2,4,6-trimethyl-2,4,6-trivinylcyclotrisiloxane and 2,4,6,8-tetramethyl-2,4,6,8-tetravinylcyclotetrasiloxane; vinyl-containing branched polysiloxanes, methylvinylsilicone resins, and methylvinylphenylsilicone resins.

The hydrosilation reaction is carried out in the presence of a catalyst. Examples of the catalyst which can be used herein include platinum group metal elements such as platinum (inclusive of platinum black), rhodium and palladium; platinum chloride, chloroplatinic acid and chloroplatinic acid salts such as H₂PtCl₄·xH₂O, H₂PtCl₆·xH₂O, NaHPtCl₆·xH₂O, KHPtCl₆·xH₂O, Na₂PtCl₆·xH₂O, K₂PtCl₄·xH₂O, PtCl₄·xH₂O, PtCl₂, and Na₂HPtCl₄·xH₂O wherein x is preferably an integer of 0 to 6, more preferably 0 or 6; alcohol-modified chloroplatinic acid as described in USP 3,220,972; complexes of chloroplatinic acid with olefins as described in USP 3,159,601, USP 3,159,662 and USP 3,775,452; platinum group metals such as platinum black and palladium on supports such as alumina, silica and carbon; rhodium-olefin complexes; chlorotris(triphenylphosphine)rhodium, known as Wilkinson catalyst; and complexes of platinum chloride, chloroplatinic acid or chloroplatinic acid salts with vinyl-containing siloxanes (specifically, vinyl-containing cyclic siloxanes).

The catalyst is used in a catalytic amount. In most cases, the amount of the catalyst is preferably 0.001 to 0.1% by weight of platinum group metal based on the total amount of the reaction polymer. In the polymerization reaction, a solvent may be used if necessary. Preferred examples of the solvent include hydrocarbon solvents such as toluene and xylene. The amount of the solvent, though not particularly limited, is preferably 30 to 1,000 parts by weight (i.e., 0.3 to 10 times greater) per 100 parts by weight of the fluorene compound and the SiH-containing organosilicon compound combined. Since the polymerization conditions are normally set such that the catalyst may not be deactivated and polymerization be completed in a short time, the polymerization temperature is, for example, 40 to 150°C, preferably 60 to 120°C. As to the polymerization time which varies with the type and amount of the polymer, the polymerization is preferably completed within 0.5 to 100 hours, especially 0.5 to 30 hours in order to avoid the entry of moisture into the polymerization system. At the end of polymerization reaction, the solvent if any is distilled off, obtaining the silicon-containing fluorene compound.

The fluorene compound having an electron-attractive substituent exhibits higher oxidation resistance than prior art fluorene compounds having allyl groups and excellent light resistance and is useful in the synthesis of fluorene skeleton-containing organosilicon compounds.

### EXAMPLES

Examples and Comparative Examples are given below for illustrating the invention although the invention is not limited thereto. In Examples and Comparative Examples, Mw is measured by gel permeation chromatography (GPC) versus monodisperse polystyrene standards using a GPC column TSKGEL Super HZM-H (Tosoh Corp.) under analysis conditions, flow rate: 0.6 mL/min, eluent: tetrahydrofuran, and column temperature: 40°C.

The compounds used in synthesis are identified below.

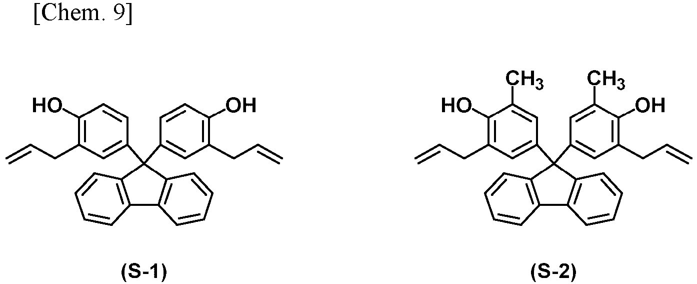

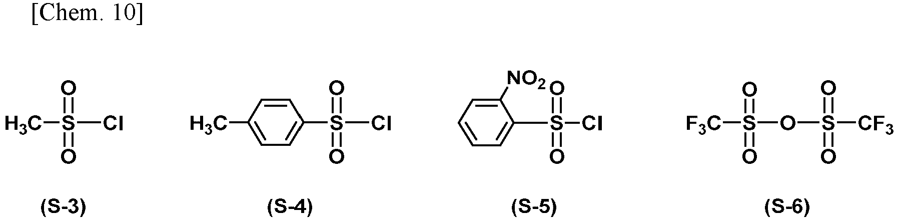

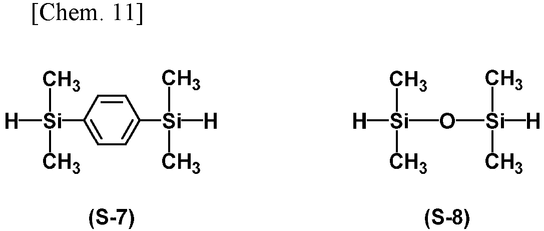

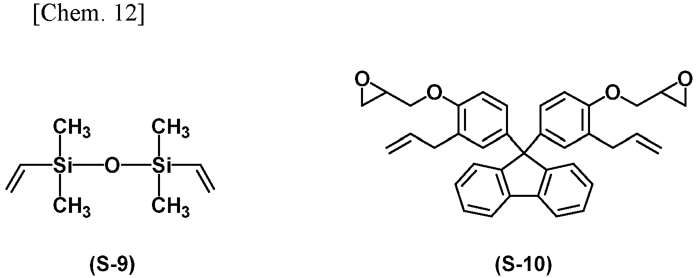

### [Example 1]

A 2-L flask equipped with a thermometer, nitrogen gas inlet tube, reflux condenser, and dropping funnel was charged with 215.3 g (0.50 mol) of the compound having formula (S-1) and 202.4 g (2.0 mol) of triethylamine. Then 350 g of methylene chloride was added to the mixture, which was stirred and cooled at 0°C. Thereafter, through the dropping funnel, 229.1 g (2.0 mol) of the compound having formula (S-3) was added dropwise over one hour. At the end of addition, the reaction solution was heated at 70°C and stirred for 8 hours, and washed with an aqueous solution of sodium carbonate and deionized water. The solvent was distilled off under reduced pressure, obtaining a solid. By UV/Vis absorption spectroscopy and ¹H-NMR spectroscopy (Bruker Corp.), the solid was identified to be a compound having the following formula (5).

¹H-NMR (CDCl₃): δ = 3.02 (6H, s), 3.25 (4H, d, J=6.3 Hz), 4.75-4.90 (4H, m), 5.76 (2H, ddt, J=17.1, 10.6, 6.3 Hz), 6.55 (2H, d, J=1.2 Hz), 6.92 (2H, d, J=8.3 Hz), 7.12 (2H, dd, J=8.3, 1.2 Hz), 7.31-7.45 (4H, m), 7.65 (2H, dd, J=8.1, 1.4 Hz), 7.85 (2H, dd, J=8.5, 1.5 Hz).

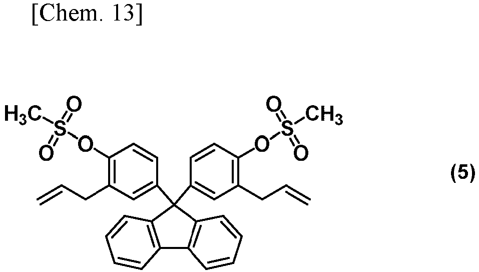

### [Example 2]

A 2-L flask equipped with a thermometer, nitrogen gas inlet tube, reflux condenser, and dropping funnel was charged with 215.3 g (0.50 mol) of the compound having formula (S-1) and 237.3 g (3.0 mol) of pyridine. Then 350 g of tetrahydrofuran was added to the mixture, which was stirred and cooled at 0°C. Thereafter, through the dropping funnel, 476.6 g (2.5 mol) of the compound having formula (S-4) in 200 g of tetrahydrofuran was added dropwise over one hour. At the end of addition, the reaction solution was heated at 90°C and stirred for 7 hours, and washed with an aqueous solution of sodium hydroxide and deionized water. The solvent was distilled off under reduced pressure, obtaining a solid. By UV/Vis absorption spectroscopy and ¹H-NMR spectroscopy (Bruker Corp.), the solid was identified to be a compound having the following formula (6).

¹H-NMR (CDCl₃): δ = 2.34 (6H, s), 3.23 (4H, d, J=6.3 Hz), 4.76-4.91 (4H, m), 5.76 (2H, ddt, J=17.1, 10.6, 6.3 Hz), 6.55 (2H, d, J=1.2 Hz), 6.87 (2H, d, J=8.4 Hz), 7.11 (2H, dd, J=8.4, 1.2 Hz), 7.28-7.45 (8H, m), 7.59-7.72 (6H, m), 7.85 (2H, dd, J=8.5, 1.5 Hz).

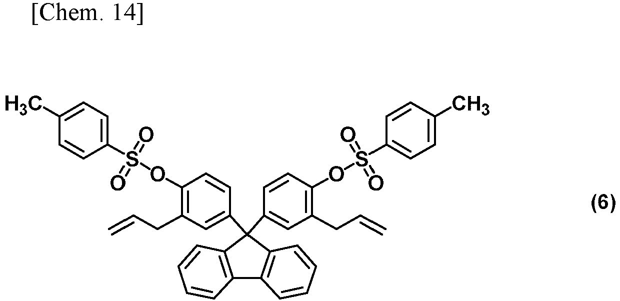

### [Example 3]

A 2-L flask equipped with a thermometer, nitrogen gas inlet tube, reflux condenser, and dropping funnel was charged with 183.4 g (0.40 mol) of the compound having formula (S-2) and 94.9 g (1.2 mol) of pyridine. Then 200 g of methylene chloride was added to the mixture, which was stirred and cooled at 0°C. Thereafter, through the dropping funnel, 221.6 g (1.0 mol) of the compound having formula (S-5) in 100 g of methylene chloride was added dropwise over one hour. At the end of addition, the reaction solution was heated at 60°C and stirred for 12 hours, and washed with an aqueous solution of potassium carbonate and deionized water. The solvent was distilled off under reduced pressure, obtaining a solid. By UV/Vis absorption spectroscopy and ¹H-NMR spectroscopy (Bruker Corp.), the solid was identified to be a compound having the following formula (7).

¹H-NMR (CDCl₃): δ = 2.31 (6H, s), 3.26 (4H, d, J=6.9 Hz), 4.75-4.90 (4H, m), 5.80 (2H, ddt, J=16.4, 10.6, 6.9 Hz), 6.80-6.91 (4H, m), 7.27-7.45 (4H, m), 7.65 (2H, dd, J=8.1, 1.4 Hz), 7.78-8.22 (10H, m).

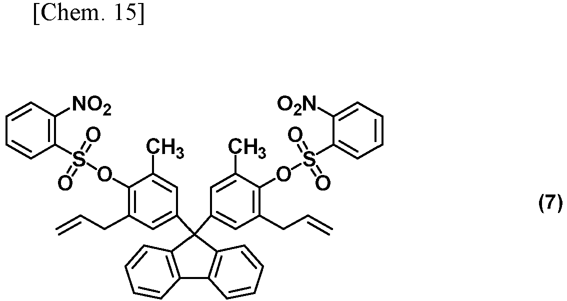

### [Example 4]

A 2-L flask equipped with a thermometer, nitrogen gas inlet tube, reflux condenser, and dropping funnel was charged with 183.4 g (0.40 mol) of the compound having formula (S-2) and 242.9 g (2.4 mol) of triethylamine. Then 400 g of tetrahydrofuran was added to the mixture, which was stirred and cooled at 0°C. Thereafter, through the dropping funnel, 338.6 g (1.2 mol) of the compound having formula (S-6) was added dropwise over one hour. At the end of addition, the reaction solution was heated at 100°C and stirred for 15 hours, and washed with an aqueous solution of sodium carbonate and deionized water. The solvent was distilled off under reduced pressure, obtaining a solid. By UV/Vis absorption spectroscopy and ¹H-NMR spectroscopy (Bruker Corp.), the solid was identified to be a compound having the following formula (8).

¹H-NMR (CDCl₃): δ = 2.31 (6H, s), 3.25 (4H, d, J=6.9 Hz), 4.76-4.90 (4H, m), 5.78 (2H, ddt, J=17.1, 10.6, 6.9 Hz), 6.81-6.91 (4H, m), 7.27-7.45 (4H, m), 7.65 (2H, dd, J=8.1, 1.4 Hz), 7.85 (2H, dd, J=8.5, 1.5 Hz).

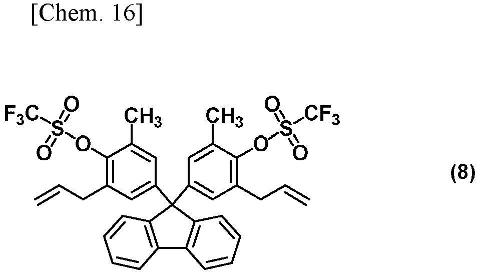

### [Example 5]

A 5-L flask equipped with a stirrer, thermometer, nitrogen purge line, and reflux condenser was charged with 587 g (1.00 mol) of the compound having formula (5) and 1,500 g of toluene and heated at 70°C. Thereafter, 1.0 g of chloroplatinic acid in toluene (Pt concentration 0.5 wt%) was added and 133 g (0.99 mol) of the compound having formula (S-8) was added dropwise over one hour (total moles of hydrosilyl group/total moles of alkenyl group = 0.99/1). At the end of addition, the reaction solution was heated at 100°C and aged for 8 hours. From the reaction solution, toluene was distilled off under reduced pressure, obtaining Resin 1. Resin 1 had a Mw of 32,000. By ¹H-NMR spectroscopy (Bruker Corp.), Resin 1 was identified to be a polymer having the following formula (9).

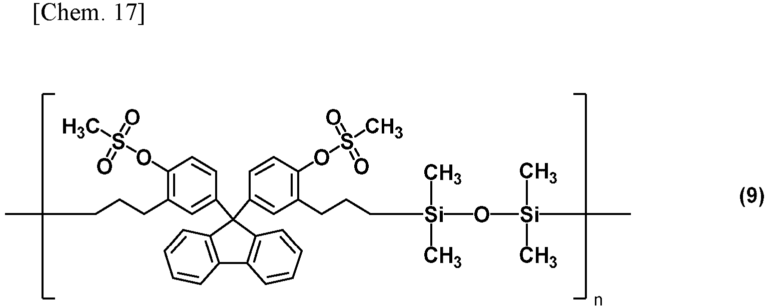

### [Example 6]

A 5-L flask equipped with a stirrer, thermometer, nitrogen purge line, and reflux condenser was charged with 739 g (1.00 mol) of the compound having formula (6) and 1,500 g of toluene and heated at 70°C. Thereafter, 1.0 g of chloroplatinic acid in toluene (Pt concentration 0.5 wt%) was added and 192 g (0.99 mol) of the compound having formula (S-7) was added dropwise over one hour (total moles of hydrosilyl group/total moles of alkenyl group = 0.99/1). At the end of addition, the reaction solution was heated at 100°C and aged for 8 hours. From the reaction solution, toluene was distilled off under reduced pressure, obtaining Resin 2. Resin 2 had a Mw of 35,000. By ¹H-NMR spectroscopy (Bruker Corp.), Resin 2 was identified to be a polymer having the following formula (10).

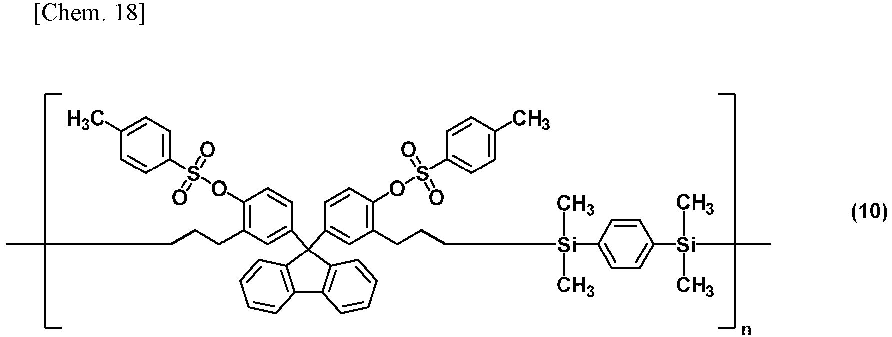

### [Example 7]

A 5-L flask equipped with a stirrer, thermometer, nitrogen purge line, and reflux condenser was charged with 580 g (0.70 mol) of the compound having formula (7), 55.9 g (0.30 mol) of the compound having formula (S-9) and 1,500 g of toluene and heated at 70°C. Thereafter, 1.0 g of chloroplatinic acid in toluene (Pt concentration 0.5 wt%) was added and 192 g (0.99 mol) of the compound having formula (S-7) was added dropwise over one hour (total moles of hydrosilyl group/total moles of alkenyl group = 0.99/1). At the end of addition, the reaction solution was heated at 100°C and aged for 8 hours. From the reaction solution, toluene was distilled off under reduced pressure, obtaining Resin 3. Resin 3 had a Mw of 29,000. By ¹H-NMR spectroscopy (Bruker Corp.), Resin 3 was identified to be a polymer having the following formula (11) wherein a=0.7 and b=0.3.

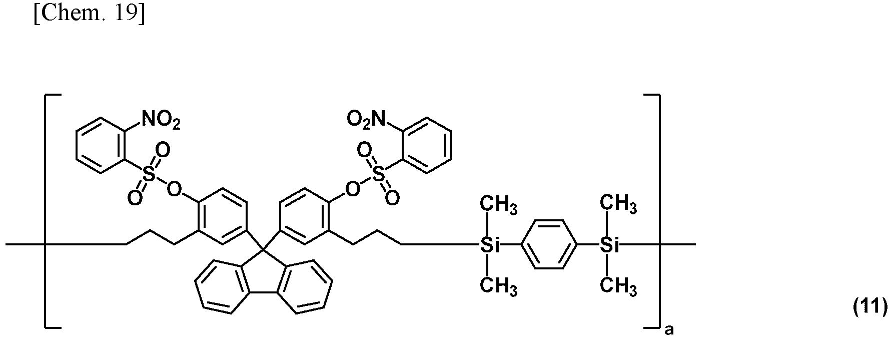

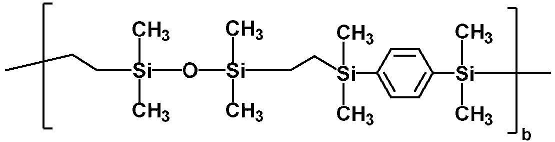

### [Example 8]

A 5-L flask equipped with a stirrer, thermometer, nitrogen purge line, and reflux condenser was charged with 578 g (0.80 mol) of the compound having formula (8), 37.3 g (0.20 mol) of the compound having formula (S-9) and 1,500 g of toluene and heated at 70°C. Thereafter, 1.0 g of chloroplatinic acid in toluene (Pt concentration 0.5 wt%) was added and 133 g (0.99 mol) of the compound having formula (S-8) was added dropwise over one hour (total moles of hydrosilyl group/total moles of alkenyl group = 0.99/1). At the end of addition, the reaction solution was heated at 100°C and aged for 8 hours. From the reaction solution, toluene was distilled off under reduced pressure, obtaining Resin 4. Resin 4 had a Mw of 26,000. By ¹H-NMR spectroscopy (Bruker Corp.), Resin 4 was identified to be a polymer having the following formula (12) wherein a=0.8 and b=0.2.

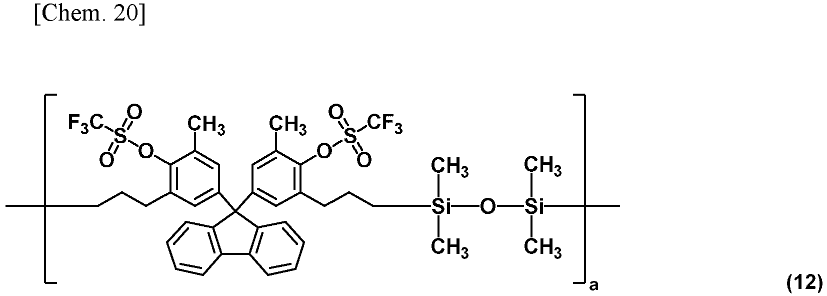

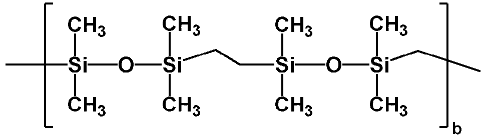

### [Comparative Example 1]

A 5-L flask equipped with a stirrer, thermometer, nitrogen purge line, and reflux condenser was charged with 431 g (1.00 mol) of the compound having formula (S-1) and 1,500 g of toluene and heated at 70°C. Thereafter, 1.0 g of chloroplatinic acid in toluene (Pt concentration 0.5 wt%) was added and 133 g (0.99 mol) of the compound having formula (S-8) was added dropwise over one hour (total moles of hydrosilyl group/total moles of alkenyl group = 0.99/1). At the end of addition, the reaction solution was heated at 100°C and aged for 8 hours. From the reaction solution, toluene was distilled off under reduced pressure, obtaining Comparative Resin 1. Comparative Resin 1 had a Mw of 29,000. By ¹H-NMR spectroscopy (Bruker Corp.), Comparative Resin 1 was identified to be a polymer having the following formula (13).

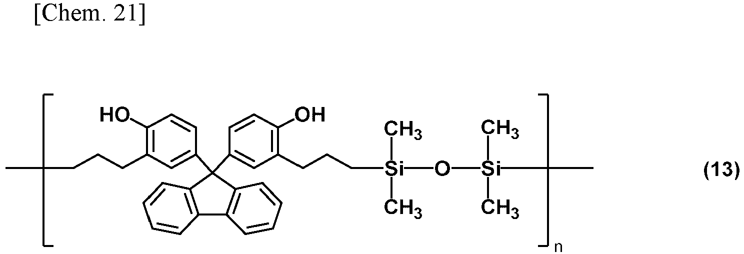

### [Comparative Example 2]

A 5-L flask equipped with a stirrer, thermometer, nitrogen purge line, and reflux condenser was charged with 543 g (1.00 mol) of the compound having formula (S-10) and 1,500 g of toluene and heated at 70°C. Thereafter, 1.0 g of chloroplatinic acid in toluene (Pt concentration 0.5 wt%) was added and 192 g (0.99 mol) of the compound having formula (S-7) was added dropwise over one hour (total moles of hydrosilyl group/total moles of alkenyl group = 0.99/1). At the end of addition, the reaction solution was heated at 100°C and aged for 9 hours. From the reaction solution, toluene was distilled off under reduced pressure, obtaining Comparative Resin 2. Comparative Resin 2 had a Mw of 33,000. By ¹H-NMR spectroscopy (Bruker Corp.), Comparative Resin 2 was identified to be a polymer having the following formula (14).

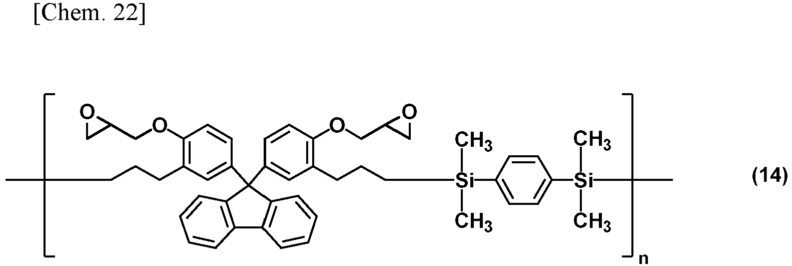

### [Light transmittance test 1]

Each of Resins 1 to 4 and Comparative Resins 1 and 2 was dissolved in cyclopentanone to form a resin solution having a concentration of 50% by weight. The resin solution was coated onto a glass substrate, heated at a temperature of 60°C for 30 minutes, and heated in nitrogen atmosphere at a temperature of 190°C for 2 hours to form a resin film of 10 µm thick. Using a spectrophotometer U-3900H (Hitachi High-Tech Corp.), the film was measured for transmittance of light of wavelength 400 nm. The results are shown in Table 1.

**[Table 1]**

| | Resin | | | | Comparative Resin | |
|---|---|---|---|---|---|---|
| | 1 | 2 | 3 | 4 | 1 | 2 |
| Light transmittance (%, 400 nm) | 96 | 98 | 96 | 97 | 96 | 97 |

### [Light transmittance test 2]

In an oven at 50°C, the film/glass wafer sample prepared above was continuously irradiated with laser light of 400 nm and 1 W. Using a spectrophotometer U-3900H (Hitachi High-Tech Corp.), the film was measured for transmittance of light of wavelength 400 nm at the lapse of 100 hours and 1,000 hours. The results are shown in Table 2.

**[Table 2]**

| | Laser irradiation time | Resin | | | | Comparative Resin | |
|---|---|---|---|---|---|---|---|
| | | 1 | 2 | 3 | 4 | 1 | 2 |
| Light transmittance (%, 400 nm) | 100 hr | 96 | 97 | 96 | 97 | 72 | 73 |
| | 1,000 hr | 96 | 97 | 95 | 96 | 62 | 61 |

It is seen from the above results that fluorene compounds having sulfonic acid ester structures and allyl groups can be synthesized according to the invention. Using the fluorene compounds, fluorene skeleton-containing organosilicon compounds or polymers having sulfonic acid ester structures as exemplified by Resins 1 to 4 can be synthesized. The films thereof undergo little oxidation over a long time. That is, films having high light resistance are provided.

## Claims

1. A fluorene compound having sulfonic acid ester structures and allyl groups, represented by the formula (1): 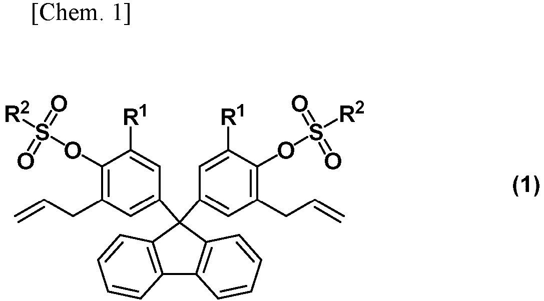 wherein R¹ is each independently hydrogen or methyl and
R² is each independently a C1-C20 hydrocarbyl group in which some or all of the hydrogen atoms may be substituted by halogen, nitro or cyano, and an ester bond or ether bond may intervene in a carbon-carbon bond.

2. The fluorene compound of claim 1 wherein both R¹ are hydrogen.

3. The fluorene compound of claim 1 or 2 wherein both R² have 1 to 8 carbon atoms.

4. A method for preparing a fluorene compound represented by the formula (1), comprising the step of reacting a fluorene compound having allyl groups, represented by the formula (2), with a compound represented by the formula (3) or (4), 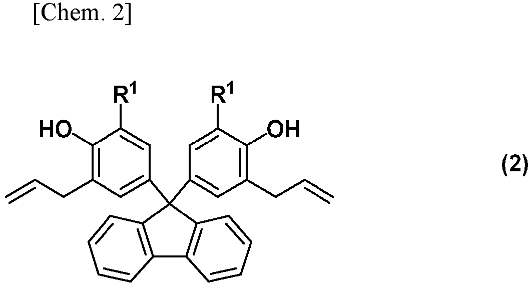 wherein R¹ is each independently hydrogen or methyl, 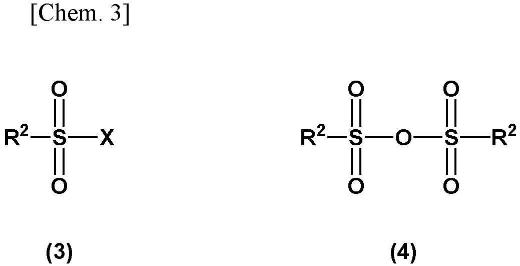 wherein R² is each independently a C1-C20 hydrocarbyl group in which some or all of the hydrogen atoms may be substituted by halogen, nitro or cyano, and an ester bond or ether bond may intervene in a carbon-carbon bond, and X is fluorine, chlorine or bromine, 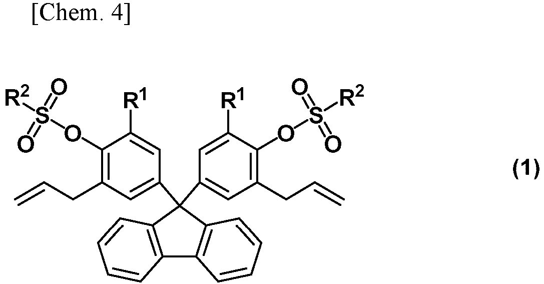 wherein R¹ and R² are as defined above.

5. A method for preparing a fluorene skeleton-containing organosilicon compound comprising the step of effecting hydrosilation reaction of the fluorene compound of any one of claims 1 to 3 with an organosilicon compound containing a Si-H group in the presence of a catalyst.
